(19)

**Europäisches Patentamt**
**European Patent Office**
**Office européen des brevets**

(11) **EP 4 582 457 A1**

(12) **EUROPEAN PATENT APPLICATION**
published in accordance with Art. 153(4) EPC

(43) Date of publication:
**09.07.2025 Bulletin 2025/28**

(21) Application number: **23860445.8**

(22) Date of filing: **30.08.2023**

(51) International Patent Classification (IPC):
*C07K 19/00* (2006.01)   *A61K 38/00* (2006.01)
*A61K 47/68* (2017.01)   *A61P 1/16* (2006.01)
*A61P 3/06* (2006.01)   *A61P 9/00* (2006.01)
*A61P 9/10* (2006.01)   *A61P 9/14* (2006.01)
*A61P 11/00* (2006.01)   *A61P 11/06* (2006.01)
*A61P 13/12* (2006.01)   *A61P 17/00* (2006.01)
*A61P 21/00* (2006.01)   *A61P 37/06* (2006.01)
*C07K 7/08* (2006.01)   *C07K 16/00* (2006.01)

(52) Cooperative Patent Classification (CPC):
**A61K 38/00; A61K 47/68; A61P 1/16; A61P 3/06;**
**A61P 9/00; A61P 9/10; A61P 9/14; A61P 11/00;**
**A61P 11/06; A61P 13/12; A61P 17/00; A61P 21/00;**
**A61P 37/06; C07K 7/08; C07K 16/00;**        (Cont.)

(86) International application number:
**PCT/JP2023/031626**

(87) International publication number:
**WO 2024/048683 (07.03.2024 Gazette 2024/10)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **30.08.2022   JP 2022137338**

(71) Applicants:
• **Mirabiologics Inc.**
  **Tokyo 153-0041 (JP)**
• **National University Corporation Kanazawa**
  **University**
  **Ishikawa 920-1192 (JP)**

(72) Inventors:
• **SAKO, Yusuke**
  **Tokyo 153-0041 (JP)**
• **HIRAI, Hidenori**
  **Tokyo 153-0041 (JP)**
• **MATSUMOTO, Kunio**
  **Kanazawa-shi, Ishikawa 920-1192 (JP)**
• **SAKAI, Katsuya**
  **Kanazawa-shi, Ishikawa 920-1192 (JP)**

(74) Representative: **D Young & Co LLP**
  **3 Noble Street**
  **London EC2V 7BQ (GB)**

Remarks:
The complete document including Reference
Table(s) and the Sequence Listing(s) can be
downloaded from the EPO website

(54) **ARTIFICIAL PROTEIN AND PHARMACEUTICAL COMPOSITION**

(57)    The present invention provides a novel substance that suitably functions as a Met agonist. The present invention provides an artificial protein that contains an Fc region of an antibody, where an amino acid sequence of SEQ ID NO: 1 is inserted into the Fc region (in the SEQ ID NO: 1, $X^1$, $X^2$, $X^3$, $X^4$, $X^5$, $X^6$, $X^7$, $X^8$, $X^9$, $X^{10}$, and $X^{11}$ each independently represent an amino acid, and n1 and n2 are each independently an integer of 0 or more and 6 or less).
$(X^1)_{n1}YX^3X^4FYYNYX^5X^6X^7WX^8X^9X^{10}X^{11}(X^2)_{n2}$   (SEQ ID NO: 1)

**(Cont. next page)**

EP 4 582 457 A1

(52) Cooperative Patent Classification (CPC): (Cont.)
**C07K 19/00**

## Description

## Technical Field

The present invention relates to an artificial protein and a pharmaceutical composition.

## Background Art

Although maintenance and recovery of liver function contribute to treatment of hepatitis, there is currently no treatment that is extremely effective in maintaining and recovering liver function, and it is particularly difficult to ameliorate liver fibrosis. The fact that, regarding hepatocytes, astrocytes, and liver macrophages, in each of the cell-specific Met (hepatocyte growth factor (HGF) receptor) deficient mice, the pathological condition of hepatitis is aggravated in any of the mice, whereas HGF exhibits regeneration promotion, inflammation suppression, and fibrosis amelioration through Met activation, which has been shown in various liver disease models. It has been known that Met-HGF is centrally involved in morphogenesis in development, wound healing, and maintenance of organ homeostasis by stimulating cell growth, survival, and migration and that a recombinant HGF protein exhibits a therapeutic effect in a preclinical model and a patient having a specific disease (Non-Patent Literatures 1 to 20).

## Citation List

Non-Patent Literature 1: Trusolino, L., Bertotti, A., & Comoglio, P.M. MET signalling: principles and functions in development, organ regeneration and cancer. Nat. Rev. Mol. Cell. Biol. 11, 834-848 (2010).
Non-Patent Literature 2: Sakai, K., Aoki, S., & Matsumoto, K. Hepatocyte growth factor and Met in drug discovery. J. Biochem. 157, 271-284 (2015).
Non-Patent Literature 3: Desole, C. et al. HGF and MET: From brain development to neurological disorders. Front Cell Dev Biol. 9, 683609 (2021).
Non-Patent Literature 4: Hirano, S. et al. A phase I/II exploratory clinical trial for intracordal injection of recombinant hepatocyte growth factor for vocal fold scar and sulcus. J Tissue Eng Regen Med. 12, 1031-1038 (2018).
Non-Patent Literature 5: Nagoshi, N. et al. Phase I/II Study of intrathecal administration of recombinant human hepatocyte growth factor in patients with acute spinal cord injury: A double-blind, randomized clinical trial of safety and efficacy. J Neurotrauma. 37, 1752-1758 (2020).
Non-Patent Literature 6: Ido, A. et al. Safety and pharmacokinetics of recombinant human hepatocyte growth factor (rh-HGF) in patients with fulminant hepatitis: a phase I/II clinical trial, following preclinical studies to ensure safety. J Transl Med. 9, 55 (2011).
Non-Patent Literature 7: Li, N. et al. Therapeutic effect of HGF on NASH mice through HGF/c-Met and JAK2-STAT3 signalling pathway. Ann Hepatol. 17, 501-510 (2018)
Non-Patent Literature 8: Yang, Y.M. et al. Interventional potential of recombinant feline hepatocyte growth factor in a mouse model of non-alcoholic steatohepatitis. Front Endocrinol. 9, 378 (2018)
Non-Patent Literature 9: Matsuda, Y. et al. Hepatocyte growth factor prevents liver cirrhosis caused by dimethylnitrosamine in rats. J Biochem. 118, 643-649 (1995).
Non-Patent Literature 10: Mizuno, S. et al. Hepatocyte growth factor prevents renal fibrosis and dysfunction in a mouse model of chronic renal disease. J Clin Invest. 101, 1827-1834 (1998).
Non-Patent Literature 11: Kawaida, K. et al. Hepatocyte growth factor prevents acute renal failure and accelerate renal regeneration in mice. Proc Natl Acad Sci USA. 91, 4357-4361 (1994).
Non-Patent Literature 12: Kosai, K. Hepatocyte growth factor abrogates Fas-induced lethal liver apoptosis in mice. Biochem Biophys Res Commun. 244: 683-690 (1998).
Non-Patent Literature 13: Tahara, M.et al. Hepatocyte growth factor leads to recovery from alcohol-induced fatty liver in rats. J Clin Invest. 103: 313-320 (1999).
Non-Patent Literature 14: Yaekashiwa, M. et al. Simultaneous or delayed administration of hepatocyte growth factor (HGF) equally repress the fibrotic change in murine lung injury induced by bleomycin: A morphologic study. Am J Resp & Crit Care Med. 156: 1937-1944 (1997).
Non-Patent Literature 15: Nakamura, T. et al. Hepatocyte growth factor prevents tissue fibrosis, remodeling, and dysfunction in cardiomyopathic hamster hearts. Am J Physiol. 288: H2131-H2139 (2005).
Non-Patent Literature 16: Azuma, H. et al. Hepatocyte growth factor prevents development of chronic allograft nephropathy in an experimental rat transplant model. J Am Soc Nephrol. 12: 1280-1292 (2001).
Non-Patent Literature 17: Ito, W. et al. Growth factors temporally associate with airway responsiveness and inflammation in allergen-exposed mice. Int Arch Allergy Immunol. 145, 324-339 (2008).

Non-Patent Literature 18: Ohmichi, H. Matsumoto, K. & Nakamura, T. In vivo mitogenic action of hepatocyte growth factor on lung epithelial cells: pulmotrophic role in lung regeneration. Am J Physiol. 270, L1031-L1039 (1996).

Non-Patent Literature 19: Date, I. et al. Hepatocyte growth factor attenuates cerebral ischemia-induced learning dysfunction. Biochem Biophys Res Commun. 319, 1152-1158 (2004).

Non-Patent Literature 20: Inampudi, C. et al. Angiogenesis in peripheral arterial disease. Curr Opin Pharmacol. 39, 60-67 (2018).

**Summary of Invention**

**Technical Problem**

However, although some cytokines and cell growth factors, which are not limited to HGF, are used as pharmaceutical drugs, it is difficult to develop a technique that leads to the amelioration of physicochemical stability and/or drug pharmacokinetics, particularly the achievement of a significant extension of half-life and/or the achievement of blood-brain barrier permeability. As a result, it is difficult to apply HGF to a therapeutic drug, including a therapeutic drug for chronic hepatitis.

Therefore, an object of the present invention is to provide a novel substance that suitably functions as a Met agonist.

**Solution to Problem**

As a result of intensive studies to achieve the above object, the inventors of the present invention have found that an artificial protein having a portion in which a specific amino acid sequence is inserted into an Fc region of an antibody functions suitably as a Met agonist, thereby completing the present invention.

That is, the present invention is as follows.

[1] An artificial protein that contains an Fc region of an antibody, where an amino acid sequence set forth in SEQ ID NO: 1 is inserted into the Fc region,

$(X^1)_{n1}YX^3X^4FYYNYX^5X^6X^7WX^8X^9X^{10}X^{11}(X^2)_{n2}$ (SEQ ID NO: 1) (in the SEQ ID NO: 1, $X^1$, $X^2$, $X^3$, $X^4$, $X^5$, $X^6$, $X^7$, $X^8$, $X^9$, $X^{10}$, and $X^{11}$ each independently represent an amino acid, and n1 and n2 are each independently an integer of 0 or more and 6 or less).

[2] The artificial protein according to [1], in which the amino acid sequence set forth in the SEQ ID NO: 1 is inserted into a loop portion, wherein the loop portion is exposed on a molecular surface of the Fc region of the antibody.

[2-1] The artificial protein according to [1], in which an amino acid sequence set forth in the SEQ ID NO: 1 is inserted into the following (10) or (11).

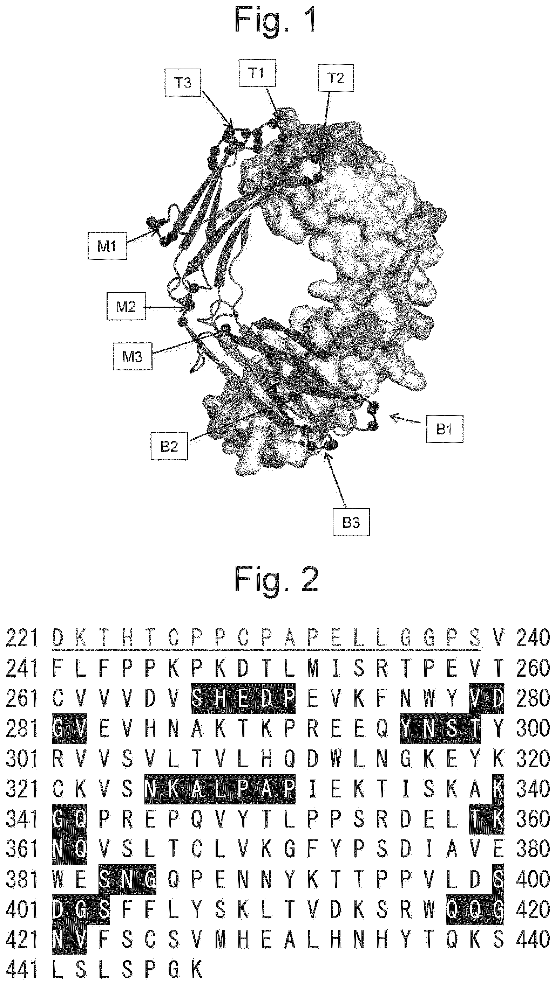

(10) Portions indicated as T1 to T3, M1 to M3, and B1 to B3 in Figure 1 in a case where the Fc region of the antibody is set forth in SEQ ID NO: 2, 3, 4, or 12.

(11) Portions corresponding to portions indicated as T1 to T3, M1 to M3, and B1 to B3 in Figure 1, in a case where the Fc region of the antibody has an amino acid sequence other than SEQ ID NO: 2, 3, 4, or 12, when the amino acid sequence of the Fc region is aligned with the amino acid sequence set forth in the SEQ ID NO: 4.

[3] The artificial protein according to any one of [1], [2], and [2-1], in which the amino acid sequence inserted into the Fc region is set forth in SEQ ID NO: 28,

$(X^1)_{n1}YPTFYYNYRSEWVLTS(X^2)_{n2}$ (SEQ ID NO: 28) (in the SEQ ID NO: 28, $X^1$, $X^2$, n1, and n2 are each defined in the same manner as in the SEQ ID NO: 1).

[4] The artificial protein according to any one of [1], [2], [2-1], and [3], in which the Fc region of the antibody is derived from an Fc region of IgG.

[4-1] The artificial protein according to any one of [1], [2], [2-1], or [3], in which the Fc region of the antibody is an Fc region of an antibody derived from any one of a human, a mouse, a rat, a rabbit, a horse, or a dog, and preferably an Fc region of an antibody derived from a human.

[4-2] The artificial protein according to any one of [1], [2], [2-1], [3], or [4-1], in which the Fc region of the antibody is an Fc region of an antibody derived from any one of IgG, IgM, IgA, IgD, or IgE.

[5] The artificial protein according to any one of [4], [4-1], or [4-2], in which the Fc region of the antibody is derived from an Fc region of IgG1 or IgG4, and preferably derived from an Fc region of IgG1.

[6] The artificial protein according to any one of [1] to [5], [2-1], and [4-1] to [4-2], in which the Fc region of the antibody is an Fc region of an antibody in which one or more amino acids are deleted, added, and/or substituted compared to an Fc region of a wild-type antibody.

[6-1] The artificial protein according to any one of [1] to [5], [2-1], and [4-1] to [4-2], in which the Fc region of the antibody is an Fc region of an antibody in which one or more amino acids are deleted, added, and/or substituted compared to an Fc region of a wild-type antibody so that binding to a Fc receptor and/or effector function of the Fc region are reduced or deleted.

[7] A Met protein agonist including:
the artificial protein according to any one of [1] to [6], [2-1], [4-1], [4-2], or [6-1].

[8] A pharmaceutical composition including:
the artificial protein according to any one of [1] to [6], [2-1], [4-1], [4-2], or [6-1].

[9] The pharmaceutical composition according to [8], in which the pharmaceutical composition is used for treatment or prevention of a disease selected from the group consisting of acute hepatitis, fulminant hepatitis, hepatic cirrhosis, hepatic fibrosis, biliary atresia, fatty liver, non-alcoholic steatohepatitis, alcoholic steatohepatitis, acute renal failure, chronic kidney disease, diabetic nephropathy, acute pneumonia, pulmonary fibrosis, angiopathy, myocardial infarction, dilated cardiomyopathy, skin ulcer, cerebral infarction, amyotrophic lateral sclerosis, glomerular nephritis, chronic allograft nephropathy, obstructive arteriosclerosis, critical limb ischemia, pulmonary emphysema, asthma, and vocal cord scarring.

[10] A method for treating a disease, including:
administering an effective amount for treatment of the artificial protein according to any one of [1] to [6], [2-1], [4-1] to [4-2], and [6-1] to a patient in need of the artificial protein.

[11] The artificial protein according to any one of [1] to [6], [2-1], [4-1] to [4-2], and [6-1] for use in the treatment or prevention of a disease.

[12] Use of the artificial protein according to any one of [1] to [6], [2-1], [4-1] to [4-2], and [6-1] for the manufacture of a pharmaceutical composition that is used for treatment or prevention of a disease.

[13] A therapeutic or prophylactic agent for a disease, including:
the artificial protein according to any one of [1] to [6], [2-1], [4-1] to [4-2], or [6-1].

Here, in [10] to [13], the disease is preferably a disease selected from the group consisting of acute hepatitis, fulminant hepatitis, hepatic cirrhosis, hepatic fibrosis, biliary atresia, fatty liver, non-alcoholic steatohepatitis, alcoholic steatohepatitis, acute renal failure, chronic kidney disease, diabetic nephropathy, acute pneumonia, pulmonary fibrosis, angiopathy, myocardial infarction, dilated cardiomyopathy, skin ulcer, cerebral infarction, amyotrophic lateral sclerosis, glomerular nephritis, chronic allograft nephropathy, obstructive arteriosclerosis, critical limb ischemia, pulmonary emphysema, asthma, and vocal cord scarring.

**Advantageous Effect of Invention**

According to the present invention, it is possible to provide a novel substance that suitably functions as a Met agonist.

**Brief Description of Drawings**

[Figure 1] Figure 1 illustrates a three-dimensional structure of an Fc region (PDB ID: 1FC1) derived from human IgG. One heavy chain constituting the Fc region is shown by a surface model (on the back side), and the other heavy chain is shown by a ribbon model (on the front side). The loop portion exposed on the molecular surface is shown as nine black Cα spheres in the heavy chain shown by the ribbon model.

[Figure 2] Figure 2 illustrates amino acid sequences of the hinge region and the Fc region of human IgG1 (SEQ ID NO: 3). A loop structure suitable for inserting an insertion sequence is shown on a black background. The amino acid number was assigned according to Chothia et al. The portion indicated by the underline indicates a hinge region that connects an Fc region and an Fab region in the IgG molecule.

[Figure 3] Figure 3 illustrates a relationship between a Met phosphorylation level and a protein concentration in each artificial protein.

[Figure 4] Figure 4 illustrates the results of alanine scanning in the artificial protein.

[Figure 5] Figure 5 illustrates the results of deep mutational scanning of the artificial protein with respect to human Met.

[Figure 6] Figure 6 illustrates a relationship between a Met phosphorylation level and an antibody concentration or an artificial protein concentration in each artificial antibody or artificial protein.

**Description of Embodiments**

Hereinafter, embodiments (hereinafter, referred to as "the present embodiments") for carrying out the present invention will be described in detail. However, the present invention is not limited thereto, and various modifications are possible without departing from the gist of the present invention.

[Artificial protein]

The artificial protein according to the present invention includes an Fc region of an antibody, and an amino acid sequence (hereinafter, also referred to as an "insertion sequence") of SEQ ID NO: 1 is inserted into the Fc region.

$(X^1)_{n1}YX^3X^4FYYNYX^5X^6X^7WX^8X^9X^{10}X^{11}(X^2)_{n2}$ (SEQ ID NO: 1)

Here, in the SEQ ID NO: 1 described above, $X^1$, $X^2$, $X^3$, $X^4$, $X^5$, $X^6$, $X^7$, $X^8$, $X^9$, $X^{10}$, and $X^{11}$ each independently represent an amino acid, and n1 and n2 are each independently an integer of 0 or more and 6 or less.

In the present specification, the term "insertion" or "insert" is used as a term to refer to that, in an amino acid sequence of a protein before an insertion sequence is inserted, an insertion sequence appears as if the insertion sequence is inserted into the amino acid sequence.

As a result, in the artificial protein according to the present invention, a specific site in an amino acid sequence of a protein before the insertion sequence is inserted is cleaved and the two cleavage fragments are bonded via the insertion sequence such that the insertion sequence links the two cleavage fragments to each other, or a partial sequence in an amino acid sequence of a protein before the insertion is deleted and the insertion sequence is bonded to the protein such that the insertion sequence replaces the deleted partial sequence.

The artificial protein according to the present invention is an artificial protein obtained by inserting the above-described insertion sequence into an Fc region of a protein having the Fc region of an antibody (hereinafter, also referred to as a "scaffold protein").

(Scaffold protein)

In the artificial protein according to the present invention, the scaffold protein into which the insertion sequence is inserted is not particularly limited as long as it contains the Fc region of the antibody, and examples thereof include an Fc fragment of an antibody, a protein containing an Fc fragment of an antibody, a protein containing an Fc region and a hinge region of an antibody, a protein containing an Fc region and an antigen binding domain of an antibody, and an antibody. The scaffold protein may be a wild-type protein or may be an artificial protein that has been artificially modified.

The scaffold protein may be a protein consisting of an Fc fragment of an antibody, and an Fc region and a hinge region of an antibody, or an antibody, which may be a wild type or may be artificially modified.

That is, the amino acid sequence in the artificial protein according to the present invention has at least an amino acid sequence corresponding to the Fc region of the antibody and also has an insertion sequence.

In the amino acid sequence in the artificial protein according to the present invention, the amino acid sequence other than the insertion sequence is not particularly limited as long as it is an amino acid sequence derived from an amino acid sequence containing an Fc region of an antibody, and any amino acid sequence corresponding to the Fc region can be used.

In the present specification, the term "Fc region" is a region including a heavy chain constant region 2 ($C_H2$) and a heavy chain constant region 3 ($C_H3$) of an antibody. The Fc region may be any region including $C_H2$ and $C_H3$, and it may be, for example, a region consisting of a fragment on the C-terminal side, which is obtained in a case where an antibody is digested with papain or may be an artificial protein containing $C_H2$ and $C_H3$, such as a single chain Fc (scFc). In addition, the term "Fc fragment" means a protein consisting of an Fc region.

In the present specification, the term "antigen binding domain" refers to a portion of an antibody, which specifically binds to a part or the whole of an antigen. The antigen binding domain may include one or a plurality of variable regions of antibodies and may include a light chain variable region ($V_L$) and a heavy chain variable region ($V_H$). Examples of the antigen binding domain include Fv, Fab, Fab', F(ab')2, single chain Fv (scFv), single chain Fv 2 (scFv2), Diabody, and taFv.

The Fc region of the scaffold protein is not particularly limited; however, it is preferably derived from any one of human, mouse, rat, rabbit, horse, or dog, and more preferably derived from human. In addition, although the Fc region may be derived from any one of IgG, IgM, IgA, IgD, or IgE, it is preferably derived from IgG and more preferably derived from human IgG.

A large number of subclasses of IgG are known. As the subclass of IgG, for example, four kinds of subclasses of IgG1, IgG2, IgG3, and IgG4 are known in humans, four kinds of subclasses of IgG1, IgG2a, IgG2b, and IgG3 are known in mice, and four kinds of subclasses of IgG1, IgG2a, IgG2b, and IgG2c are known in rats; however, any one of these may be used. The Fc region of the scaffold protein may be derived from IgG1 or IgG4, may be derived from IgG1, may be derived from human IgG1 or human IgG4, or may be derived from human IgG1.

In the present specification, "the Fc region of the scaffold protein is derived from a specific antibody" means that the Fc region has any one of the following amino acid sequences (1) to (3).

(1) An amino acid sequence of the Fc region of the specific antibody
(2) An amino acid sequence having a deletion, a substitution, and/or an addition of one or more amino acids in the amino acid sequence of (1)
(3) An amino acid sequence having homology of 80% or more, 85% or more, 90% or more, 95% or more, 98% or more, or 99% or more to the amino acid sequence of (1).

Here, the Fc region of the specific antibody may be an Fc fragment of an antibody, or may be an Fc fragment of an antibody, which has been artificially modified, in a case where the Fc fragment of the antibody has the amino acid sequence of (2) or (3) described above.

In the present specification, in a case where one or more amino acids are deleted, substituted, and/or added in an amino acid sequence, "one or more amino acids" may be, for example, 1, 2, 3, 4, 5, 6, 7, 8, 9, or 10, may be within a range of 1 to 10, or may be 1 to 9, 1 to 8, 1 to 7, 1 to 6, 1 to 5, 1 to 4, 1 to 3, or 1 or 2.

In a case where the artificial protein contains an antigen binding domain, the antigen recognized by the antigen binding domain is not particularly limited, and the antigen may be, for example, an antigen that is specifically expressed in an organ or tissue which is intended to impart targeting to the artificial protein according to the present invention or whose expression is upregulated in the organ or tissue. For example, the antigen binding domain of the artificial protein may bind to PD-L1, PD-1, TfR, or CD3. The antigen binding domain of the artificial protein may be an antigen binding domain of the antibody that is used in Examples or an antigen binding fragment thereof.

The Fc region of the antibody contained in the scaffold protein is preferably an Fc region of an antibody in which one or more amino acids are deleted, added, and/or substituted compared to an Fc region of a wild-type antibody. The deletion, addition, and/or substitution of the one or more amino acids may be a mutation that reduces or deletes the binding of the Fc receptor and/or the effector function of the Fc region, improves the binding of the Fc receptor and/or the effector function of the Fc region, extends the biological half-life, or stabilizes the antibody. Examples of such mutations include mutations disclosed in the specification of United States Patent Application No. 2012/0251531, the specification of United States Patent No. 9505826, the specification of United States Patent Application No. 2014/0056879, the specification of United States Patent Application No. 2012/0107871, and the specification of United States Patent Application No. 2019/0300621.

From the viewpoint of increasing the stability of the artificial protein in vivo and/or improving the Met agonist activity of the artificial protein, the Fc region of the antibody contained in the scaffold protein preferably contains a deletion, addition, and/or substitution of one or more amino acids so that the binding to the Fc receptor and/or the effector function of the Fc region are reduced or deleted, more preferably contains at least two substitutions corresponding to the substitution of P329G of the Fc region of the wild-type human IgG1 and the substitution of at least one further amino acid, and still more preferably contains each of substitutions corresponding to the substitutions of P329G, L234A, and L235A of the Fc region of the wild-type human IgG1. Here, in the substitutions of P329G, L234A, and L235A, the residues are numbered according to the EU index of Kabat.

The Fc region of the antibody contained in the scaffold protein preferably has any one of the following amino acid sequences (4) to (6) and (4') to (6'), and more preferably has any one of the amino acid sequences (4) to (6).

(4) An amino acid sequence of SEQ ID NO: 2
(5) An amino acid sequence having a deletion, a substitution, and/or an addition of one or more amino acids in the amino acid sequence of (4)
(6) An amino acid sequence having homology of 80% or more, 85% or more, 90% or more, 95% or more, 98% or more, or 99% or more to the amino acid sequence of (4)
(4') An amino acid sequence of SEQ ID NO: 4 (the amino acid sequence is obtained by removing the sequences from D at a position 221 to S at a position 239 from the amino acid sequence of SEQ ID NO: 3 in Figure 2)
(5') An amino acid sequence having a deletion, a substitution, and/or an addition of one or more amino acids in the amino acid sequence of (4')
(6') An amino acid sequence having homology of 80% or more, 85% or more, 90% or more, 95% or more, 98% or more, or 99% or more to the amino acid sequence of (4')

VFLFPPKPKDTLMISRTPEVTCVVVDVSHEDPEVKFNWYVDGVE
VHNAKTKPREEQYNSTYRVVSVLTVLHQDWLNGKEYKCKVSNK
ALGAPIEKTISKAKGQPREPQVYTLPPSRDELTKNQVSLTCLVKG   (SEQ ID NO: 2)
FYPSDIAVEWESNGQPENNYKTTPPVLDSDGSFFLYSKLTVDKS
RWQQGNVFSCSVMHEALHNHYTQKSLSLSPGK

In the amino acid sequences of (5) and (6) described above, it is preferable that the deletion, substitution, and/or addition of one or more amino acids is carried out in an amino acid other than G at a position 90 of SEQ ID NO: 2.

In a case where the scaffold protein is an antibody, the antibody is not particularly limited, and examples thereof include a mouse antibody, a rat antibody, a rabbit antibody, a horse antibody, a dog antibody, a chimeric antibody, a humanized antibody, and a fully human antibody. These antibodies may be wild-type proteins or may be artificial proteins that have been artificially modified.

The scaffold protein preferably has, for example, any one of the following amino acid sequences (7) to (9) and (7') to (9'), and more preferably has any one of the amino acid sequences (7) to (9).

(7) An amino acid sequence of SEQ ID NO: 12
(8) An amino acid sequence having a deletion, a substitution, and/or an addition of one or more amino acids in the amino acid sequence of (7)
(9) An amino acid sequence having homology of 80% or more, 85% or more, 90% or more, 95% or more, 98% or more, or 99% or more to the amino acid sequence of (7)
(7') An amino acid sequence of SEQ ID NO: 3 (the amino acid sequence illustrated in Figure 2)
(8') An amino acid sequence having a deletion, a substitution, and/or an addition of one or more amino acids in the amino acid sequence of (7')
(9') An amino acid sequence having homology of 80% or more, 85% or more, 90% or more, 95% or more, 98% or more, or 99% or more to the amino acid sequence of (7')

The amino acid sequence of SEQ ID NO: 12 will be described later in Examples.

In the amino acid sequences of (8) and (9) described above, it is preferable that the deletion, substitution, and/or addition of one or more amino acids is carried out in an amino acid other than As at a position 14 and a position 15 and G at a position 109 of SEQ ID NO: 12.

(Insertion of insertion sequence)

In the present invention, the insertion sequence is inserted into the Fc region of the antibody in the scaffold protein. The insertion sequence is set forth in SEQ ID NO: 1.
$(X^1)_{n1}YX^3X^4FYYNYX^5X^6X^7WX^8X^9X^{10}X^{11}(X^2)_{n2}$ (SEQ ID NO: 1)

Here, in the SEQ ID NO: 1 described above, $X^1$, $X^2$, $X^3$, $X^4$, $X^5$, $X^6$, $X^7$, $X^8$, $X^9$, $X^{10}$, and $X^{11}$ each independently represent an amino acid, and n1 and n2 are each independently an integer of 0 or more and 6 or less.

In the present specification, the term "amino acid" includes not only a natural amino acid but also an artificial amino acid variant and a derivative thereof. Examples of the amino acid include a proteinogenic L-amino acid, a non-proteinogenic amino acid, and a chemically synthesized compound having characteristics publicly known in the related art as characteristics of an amino acid.

In a case where proteinogenic amino acids are represented by a three-letter notation generally known in the related art, the proteinogenic amino acids are Arg, His, Lys, Asp, Glu, Ser, Thr, Asn, Gln, Cys, Gly, Pro, Ala, Ile, Leu, Met, Phe, Trp, Tyr, and Val. In addition, in a case where the proteinogenic amino acids are represented by one-letter notation generally known in the related art, the proteinogenic amino acids are R, H, K, D, E, S, T, N, Q, C, G, P, A, I, L, M, F, W, Y, and V.

The non-proteinogenic amino acids include natural and unnatural amino acids other than the proteinogenic amino acids.

Examples of unnatural amino acids include an amino acid ($\alpha,\alpha$-disubstituted amino acid ($\alpha$-methylalanine or the like) in which the structure of the main chain is different from the structure of the natural type, an N-alkyl amino acid, a D-amino acid, a $\beta$-amino acid, a $\gamma$-amino acid, a $\delta$-amino acid, an $\alpha$-hydroxy acid, or the like); an amino acid (norleucine, homohistidine, or the like) in which the structure of the side chain is different from the structure of the natural type; an amino acid (a "homo" amino acid, homophenylalanine, homohistidine, or the like) having an excess methylene in the side chain; and an amino acid (cysteic acid or the like) in which a carboxylic acid functional group in the side chain is substituted with a sulfonic acid group.

In SEQ ID NO: 1, the amino acid sequence portions represented by $(X^1)_{n1}$ and $(X^2)_{n2}$ correspond to a linker and may not be present in the insertion sequence. The amino acid sequence portions represented by $(X^1)_{n1}$ and $(X^2)_{n2}$ are not particularly limited as long as the insertion sequence and the scaffold protein can be linked to each other; however, it is preferable that they have a physiologically inactive amino acid sequence.

$X^1$ and $X^2$ are each independently preferably selected from Ser, Gly, and Cys, and more preferably selected from Ser and Gly.

n1 and n2 are each independently 0 or more and 6 or less, and may be 0 or more and 5 or less, 0 or more and 4 or less, 0 or more and 3 or less, 1 or more and 6 or less, 1 or more and 5 or less, 1 or more and 4 or less, 1 or more and 3 or less, 2 or more and 6 or less, 2 or more and 5 or less, 2 or more and 4 or less, or 2 or more and 3 or less.

The sum of n1 and n2 is not particularly limited as long as n1 and n2 are within the above-described range; however, it may be, for example, 0 or more and 10 or less, 0 or more and 8 or less, 2 or more and 10 or less, 2 or more and 8 or less, 4 or more and 10 or less, or 4 or more and 8 or less.

As a result, the linker may be an amino acid sequence consisting of one or more selected from the group consisting of Ser and Gly in a length of, for example, 1 or more and 6 or less, 1 or more and 5 or less, 1 or more and 4 or less, 1 or more and 3 or less, 2 or more and 6 or less, 2 or more and 5 or less, 2 or more and 4 or less, or 2 or more and 3 or less.

In SEQ ID NO: 1, $X^1$, $X^2$, $X^3$, $X^4$, $X^5$, $X^6$, $X^7$, $X^8$, $X^9$, $X^{10}$, and $X^{11}$ are each independently an amino acid, which may be a natural amino acid, and $X^3$ to $X^{11}$ are each independently the following amino acid.

$X^3$ is preferably an amino acid other than G, D, and C, and particularly preferably P.

$X^4$ is preferably an amino acid other than G, P, D, and W, and particularly preferably T.

$X^5$ is preferably an amino acid other than G, P, C, and Y, more preferably A, E, or R, and particularly preferably R.

$X^6$ is preferably an amino acid other than V, I, P, E, C, and W, more preferably G, A, N, Q, R, or S, and particularly preferably S.

$X^7$ is preferably an amino acid other than G, P, C, and W, more preferably A, L, V, I, D, E, Q, R, S, or T, and particularly preferably E.

$X^8$ is preferably an amino acid other than G, P, D, N, F, Y, and W, more preferably A, V, I, E, K, or T, and particularly preferably V.

$X^9$ is preferably an amino acid other than D, and particularly preferably L.

$X^{10}$ is preferably an amino acid other than G, P, D, N, H, K, R, and S, more preferably A, L, V, I, or T, and particularly preferably T.

$X^{11}$ is preferably an amino acid other than V and C, more preferably A, I, P, D, E, N, Q, H, K, R, S, F, Y, or W, and particularly preferably S.

The amino acid of SEQ ID NO: 1 is preferably set forth in SEQ ID NO: 28.

$(X^1)_{n1}$YPTFYYNYRSEWVLTS$(X^2)_{n2}$ (SEQ ID NO: 28)

Here, in the SEQ ID NO: 28, $X^1$, $X^2$, n1, and n2 are each defined in the same manner as in the SEQ ID NO: 1, and the same also applies to the preferred aspects thereof.

In the present invention, the site at which the insertion sequence of the scaffold protein is inserted is not particularly limited; however, it is preferably a loop portion exposed on the molecular surface of the Fc region of the antibody. The loop portion is preferably a polypeptide chain region that links two β-strands in the Fc region, where the polypeptide chain region has a structure that has a bent structure and is exposed on the surface of the antibody molecule.

In a case where the insertion sequence is inserted into the scaffold protein, the insertion sequence may be inserted between two adjacent amino acid residues of the scaffold protein; however, it is preferable that the insertion sequence is inserted between two amino acid residues of the scaffold protein, which are not adjacent to each other. "Inserting the insertion sequence between two amino acid residues of the scaffold protein, which are not adjacent to each other," means that amino acid residues between the two amino acid residues selected for inserting the insertion sequence are deleted from the scaffold protein, and the selected two amino acid residues are directly bonded to the insertion sequence. The number of amino acid residues to be deleted from the scaffold protein in order to insert the insertion sequence is not particularly limited; however, it is preferably 0 or more and 15 or less, more preferably 0 or more and 10 or less, 0 or more and 8 or less, 0 or more and 5 or less, 0 or more and 3 or less, 1 or more and 3 or less, or 1 or more and 2 or less, and still more preferably 1. "The number of amino acid residues to be deleted from the scaffold protein in order to insert the insertion sequence is one" means that an (n+1)-th amino acid residue from the N-terminal of the scaffold protein is deleted and the insertion sequence is inserted between an n-th amino acid residue and an (n+2)-th amino acid residue. Here, n is any natural number.

In a case where the insertion sequence is inserted into the scaffold protein, the bond between the insertion sequence and the scaffold protein is preferably a covalent bond.

The loop portion into which the insertion sequence is inserted is not particularly limited in the loop structure in the Fc region of the antibody, and any site in the loop structure may be selected.

Any site in the loop structure means that two amino acid residues for inserting the insertion sequence are at a site selected from within the loop structure.

The insertion site of the insertion sequence in the scaffold protein is not particularly limited; however, specific examples thereof will be described with reference to Figure 1 and Figure 2.

Figure 1 illustrates the three-dimensional structure of the Fc region derived from human IgG. One heavy chain constituting the Fc region is shown by a surface model (on the back side), and the other heavy chain constituting the Fc region is shown by a ribbon model (on the front side).

The loop portions suitably selected as the insertion site of the insertion sequence are illustrated in Figure 1. The portions indicated as T1 to T3, M1 to M3, and B1 to B3 are the corresponding portions.

Figure 2 illustrates an amino acid sequence of an Fc region derived from human IgG1, and a loop portion suitably selected as the insertion site of the insertion sequence is shown on a black background.

The loop portions that are suitably selected include the following loop portions of the Fc region derived from human IgG1, which is set forth in the SEQ ID NO: 4,

T1 (SHEDP, SEQ ID NO: 5): positions 267 to 271
T2 (YNST, SEQ ID NO: 6): positions 296 to 299
T3 (NKALPAP, SEQ ID NO: 7): positions 325 to 331
M1 (VDGV, SEQ ID NO: 8): positions 279 to 282
M2 (KGQ): positions 340 to 342
M3 (SDGS, SEQ ID NO: 9): positions 400 to 403
B1 (TKNQ, SEQ ID NO: 10): positions 359 to 362
B2 (SNG): positions 383 to 385, and
B3 (QQGNV, SEQ ID NO: 11): positions 418 to 422.

Among these, B2 and B3 are more preferable. The amino acid sequence of the SEQ ID NO: 4 is an amino acid sequence obtained by removing the sequences from D at a position 221 to S at a position 239 from the amino acid sequence of the SEQ ID NO: 3 in Figure 2.

In a case of T1 as an example, the positions 267 to 271 correspond to the positions 47 to 51 in the SEQ ID NO: 3 and correspond to the positions 28 to 32 in the SEQ ID NO: 4. That is, the portions represented by T2 to T3, M1 to M3, and B1 to B3 correspond to amino acids at positions obtained by subtracting 220 in the SEQ ID NO: 3 or correspond to amino acids at positions obtained by subtracting 239 in the SEQ ID NO: 4.

In addition, the insertion site of the insertion sequence may be a site selected from sites within the loop structure, which is indicated by the position of the above-described amino acid residue. In a case of T1 as an example, as the site selected from the sites within the loop structure, the positions 267 and 268, the positions 268 and 269, the positions 269 and 270, the positions 270 and 271, the positions 267 and 269, the positions 268 and 270, the positions 269 and 271, the positions 267 and 270, the positions 268 and 271, and the positions 267 and 271 may be selected, and an insertion sequence may be inserted between these selected amino acid residues. Here, in a case where the selected two amino acid residues are not adjacent to each other, the amino acid residues therebetween are deleted.

In a case where an antibody other than human IgG1 or human IgG1 is modified, portions corresponding to the portions indicated as T1 to T3, M1 to M3, and B1 to B3 by aligning with the amino acid sequence set forth in the SEQ ID NO: 3 or the SEQ ID NO: 4 can be used as the insertion site of the insertion sequence, and among these, portions corresponding to the portions indicated as B2 and B3 are more preferable.

Among them, in the loop portion of the Fc region derived from human IgG1, which is set forth in the SEQ ID NO: 4, a more suitable site for inserting an insertion sequence is the position between the amino acid residues marked with an asterisk (*) in the following portions:

T1 (SH*EDP): positions 267 to 271
T2 (Y*NST): positions 296 to 299
T3 (NKALP*AP): positions 325 to 331
M1 (VD*GV): positions 279 to 282
M2 (K*GQ): positions 340 to 342
M3 (SD*GS): positions 400 to 403
B1 (TK*NQ): positions 359 to 362
B2 (S*NG): positions 383 to 385, and
B3 (QQ*GNV): positions 418 to 422.

It is still more preferably the position between the amino acid residues marked with an asterisk (*) in B2 and B3. Here, in a case of inserting an insertion sequence, any amino acid residue adjacent to the amino acid residue indicated by an asterisk (*) may be deleted.

In a case where an antibody other than human IgG1 or human IgG1 is modified, portions corresponding to the portions indicated by an asterisk (*) in T1 to T3, M1 to M3, and B1 to B3 by aligning with the amino acid sequence set forth in the SEQ ID NO: 3 or SEQ ID NO: 4 can be used as the insertion site of the insertion sequence.

In the artificial protein according to the present invention, the insertion sequence may be inserted between S at a position 383 and G at a position 385 in B2 described above while deleting N at a position 384, or the insertion sequence may be inserted between Q at a position 419 and N at a position 421 in B3 described above while deleting G at a position 420.

In the artificial protein according to the present invention, it is preferable that the scaffold protein contains an Fc region of an antibody derived from any one of human, mouse, rat, rabbit, horse, or dog, an Fc region of an antibody derived from any one of IgG, IgM, IgA, IgD, or IgE, an Fc region of an antibody derived from human, an Fc region of an antibody derived from human IgG, or an Fc region of an antibody derived from human IgG1, and the site at which the insertion sequence is inserted is preferably (10) or (11) described below. In this aspect, the scaffold protein and the insertion site of the insertion sequence may include any one of the exemplified aspects described above.

(10) The portions indicated above as T1 to T3, M1 to M3, and B1 to B3 in a case where the Fc region in the scaffold protein is set forth in the SEQ ID NO: 2, 3, 4, or 12

(11) The portions corresponding to the portions indicated as T1 to T3, M1 to M3, and B1 to B3 in a case where the Fc region in the scaffold protein has an amino acid sequence other than SEQ ID NO: 2, 3, 4, or 12, when the amino acid sequence of the Fc region is aligned with the amino acid sequence set forth in the SEQ ID NO: 4

It is noted that in a case where an artificial protein contains a portion other than the Fc region as the scaffold protein, the portion of the artificial protein, which corresponds to the amino acid sequence set forth in the SEQ ID NO: 4 when the artificial protein is aligned with the amino acid sequence set forth in the SEQ ID NO: 4, may be regarded as the Fc region portion of the artificial protein. In performing the alignment, the insertion sequence is deleted from the amino acid sequence of the artificial protein. In a case of deleting the insertion sequence, the amino acid sequence portion represented by $(X^1)_{n1}$ and $(X^2)_{n2}$ in the SEQ ID NO: 1 can be appropriately adjusted to maximize homology with the amino acid sequence set forth in the SEQ ID NO: 4 according to the alignment as long as n1 and n2 are in the above ranges.

The artificial protein according to the present invention may have, for example, any one of the following amino acid sequences (12) to (17) and (12') to (17').

(12) An amino acid sequence of SEQ ID NO: 13

(13) An amino acid sequence having a deletion, a substitution, and/or an addition of one or more amino acids in the amino acid sequence of (12)

(14) An amino acid sequence having homology of 80% or more, 85% or more, 90% or more, 95% or more, 98% or more, or 99% or more to the amino acid sequence of (12)

(15) An amino acid sequence of SEQ ID NO: 14

(16) An amino acid sequence having a deletion, a substitution, and/or an addition of one or more amino acids in the amino acid sequence of (15)

(17) An amino acid sequence having homology of 80% or more, 85% or more, 90% or more, 95% or more, 98% or more, or 99% or more to the amino acid sequence of (15)

(12') An amino acid sequence of SEQ ID NO: 15

(13') An amino acid sequence having a deletion, a substitution, and/or an addition of one or more amino acids in the amino acid sequence of (12')

(14') An amino acid sequence having homology of 80% or more, 85% or more, 90% or more, 95% or more, 98% or more, or 99% or more to the amino acid sequence of (12')

(15') An amino acid sequence of SEQ ID NO: 16

(16') An amino acid sequence having a deletion, a substitution, and/or an addition of one or more amino acids in the amino acid sequence of (15')

(17') An amino acid sequence having homology of 80% or more, 85% or more, 90% or more, 95% or more, 98% or more, or 99% or more to the amino acid sequence of (15')

The amino acid sequences of SEQ ID NOs: 13, 15, and 16 will be described later in Examples.

DKTHTCPPCPAPE**AA**GGPSVFLFPPKPKDTLMISRTPEVTCVVV
DVSHEDPEVKFNWYVDGVEVHNAKTKPREEQYNSTYRVVSVLT
VLHQDWLNGKEYKCKVSNKAL**G**APIEKTISKAKGQPREPQVYTL
PPSRDELTKNQVSLTCLVKGFYPSDIAVEWESNGQPENNYKTTP    (SEQ ID NO: 14)
PVLDSDGSFFLYSKLTVDKSRWQQ**SGGYPTFYYNYRSEWVLTS**
**GGS**NVFSCSVMHEALHNHYTQKSLSLSPGK

In the amino acid sequences of (13) and (14) described above, it is preferable that the deletion, substitution, and/or addition of one or more amino acids is carried out in an amino acid other than A at a position 14 and a position 15, G at a position 109, and from S at a position 164 to S at a position 185 of SEQ ID NO: 13.

In the amino acid sequences of (16) and (17) described above, it is preferable that the deletion, substitution, and/or addition of one or more amino acids is carried out in an amino acid other than A at a position 14 and a position 15, G at a position 109, and from S at a position 200 to S at a position 221 of SEQ ID NO: 14.

In the amino acid sequences of (13') and (14') described above, it is preferable that the deletion, substitution, and/or addition of one or more amino acids is carried out in an amino acid other than from S at a position 164 to S at a position 185 of SEQ ID NO: 15.

In the amino acid sequences of (16') and (17') described above, it is preferable that the deletion, substitution, and/or addition of one or more amino acids is carried out in an amino acid other than from S at a position 200 to S at a position 221 of SEQ ID NO: 16.

The artificial protein according to the present invention may have an amino acid sequence in which the portion "SGGYPTFYYNYRSEWVLTSGGS" (SEQ ID NO: 17) is replaced with the amino acid sequence of the SEQ ID NO: 1 in any one of the amino acid sequences of (12) to (17) and (12') to (17') described above.

The artificial protein according to the present invention may have an amino acid sequence in which the portion "SGGYPTFYYNYRSEWVLTSGGS" (SEQ ID NO: 17) is replaced with the amino acid sequence of the SEQ ID NO: 28 in any one of the amino acid sequences of (12) to (17) and (12') to (17') described above.

The artificial protein according to the present invention may have an amino acid sequence in which the portion of "SGGYPTFYYNYRSEWVLTSGGS" (SEQ ID NO: 17) has been replaced with "SGGYX$^3$X$^4$FYY-NYX$^5$X$^6$X$^7$WX$^8$X$^9$X$^{10}$X$^{11}$GGS" (SEQ ID NO: 29) in any one of the above-described amino acid sequences (12) to (17) and (12') to (17') (the definitions and preferred aspects of X$^3$, X$^4$, X$^5$, X$^6$, X$^7$, X$^8$, X$^9$, X$^{10}$, and X$^{11}$ are as described above).

The artificial protein according to the present invention may be an artificial antibody in which an antigen binding domain is bonded to the fragment having any one of the above-described amino acid sequences (12) to (17) and (12') to (17') described above.

The artificial protein according to the present invention may be an artificial antibody in which an antigen binding domain is bonded to a fragment having an amino acid sequence in which the portion "SGGYPTFYYNYRSEWVLTSGGS" (SEQ ID NO: 17) is replaced with the amino acid sequence of the SEQ ID NO: 1 in any one of the amino acid sequences of (12) to (17) and (12') to (17') described above.

The artificial protein according to the present invention may be an artificial antibody in which an antigen binding domain is bonded to a fragment having an amino acid sequence in which the portion "SGGYPTFYYNYRSEWVLTSGGS" (SEQ ID NO: 17) is replaced with the amino acid sequence of the SEQ ID NO: 28 in any one of the amino acid sequences of (12) to (17) and (12') to (17') described above.

The artificial protein according to the present invention may be an artificial antibody in which an antigen binding domain is bonded to a fragment having an amino acid sequence in which the portion of "SGGYPTFYYNYRSEWVLTSGGS" (SEQ ID NO: 17) has been replaced with "SGGYX$^3$X$^4$FYYNYX$^5$X$^6$X$^7$WX$^8$X$^9$X$^{10}$X$^{11}$GGS" (SEQ ID NO: 29) in any one of the above-described amino acid sequences (12) to (17) and (12') to (17') (the definitions and preferred aspects of X$^3$, X$^4$, X$^5$, X$^6$, X$^7$, X$^8$, X$^9$, X$^{10}$, and X$^{11}$ are as described above).

The antigen binding domain in the artificial antibody described above may be an antigen binding domain of the antibody that is used in Examples or an antigen binding fragment thereof.

A method of inserting the insertion sequence into the scaffold protein is not particularly limited, and examples thereof include the methods described in International Publication No. WO2019/026920, International Publication No. WO2020/027224, and International Publication No. WO2019/216436.

In addition, the method described in Examples may be used.

[Pharmaceutical composition and the like]

The artificial protein according to the present invention can promote the autophosphorylation of the Met protein which is a hepatocyte growth factor (HGF) receptor. As a result, the artificial protein according to the present invention is useful as a Met protein agonist and a pharmaceutical composition.

In addition, it can be used in vitro or in vivo as a protective agent or a regeneration promoting agent for an organ in a case of organ transplantation.

The pharmaceutical composition according to the present invention contains the artificial protein according to the present invention as an active ingredient. Since the pharmaceutical composition according to the present invention has an HGF-like function, it is useful for the promotion of cell proliferation, promotion of cell migration, suppression of apoptosis, induction of morphogenesis, angiogenesis, and regeneration and/or protection of tissues and organs, and it is useful as a therapeutic or prophylactic agent for diseases related to these.

Examples of such diseases include diseases that can be prevented or treated with the Met protein. In addition, the disease in the present invention may be a disease for which a therapeutic effect or a preventive effect can be expected by HGF.

Examples of such diseases include diseases in the liver, the kidney, the lung, the blood vessel, the skin, the brain, the muscle, and the nerve, and among them, examples of the diseases in the liver include hepatitis and diseases caused by hepatitis.

Specific examples thereof include, but are not limited to, acute hepatitis, fulminant hepatitis, hepatic cirrhosis, hepatic fibrosis, biliary atresia, fatty liver, non-alcoholic steatohepatitis, alcoholic steatohepatitis, acute renal failure, chronic kidney disease, diabetic nephropathy, acute pneumonia, pulmonary fibrosis, angiopathy, myocardial infarction, dilated cardiomyopathy, skin ulcer, cerebral infarction, amyotrophic lateral sclerosis, glomerular nephritis, chronic allograft nephropathy, obstructive arteriosclerosis, critical limb ischemia, pulmonary emphysema, asthma, and vocal cord scarring.

The administration form of the pharmaceutical composition according to the present invention is not particularly limited, and it may be oral administration or parenteral administration. Examples of the parenteral administration include injection administration such as intramuscular injection, intravenous injection, and subcutaneous injection, transdermal administration, transmucosal administration (transnasal, transoral, transocular, transpulmonary, vaginal, or transrectal).

The active ingredient of the pharmaceutical composition may be used as it is, or the pharmaceutical composition may be formulated by adding a carrier, an excipient, and/or an additive and the like, which are pharmaceutically acceptable. Examples of the formulation form include a liquid agent (for example, an injection agent), a dispersing agent, a suspension agent, a tablet, a pill, a powder agent, a suppository, a powder, a granule agent, a granule, a capsule agent, a syrup, a troche, an inhalant, an ointment, an eye drop, a nasal drop, an ear drop, and a poultice.

The formulation can be carried out by a conventional method by using, for example, an excipient, a binder, a disintegrating agent, a lubricant, a solvent, a dissolution assisting agent, a coloring agent, a taste masking and flavoring agent, a stabilizer, an emulsifying agent, an absorption promoting agent, a surfactant, a pH adjusting agent, a preservative, and an antioxidant as appropriate.

Examples of the component to be used for formulation include, but are not limited to, purified water, saline solution, a phosphate buffer solution, dextrose, glycerol, an organic solvent that is pharmaceutically acceptable, such as ethanol, animal and vegetable oils, lactose, mannitol, glucose, sorbitol, crystalline cellulose, hydroxypropyl cellulose, starch, corn starch, anhydrous silicic acid, aluminum magnesium silicate, collagen, polyvinyl alcohol, polyvinyl pyrrolidone, a carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohol, stearyl alcohol, stearic acid, and human serum albumin.

As the absorption promoting agent that ameliorates absorption, the following substances may be used: surfactants such as polyoxyethylene lauryl ethers, sodium lauryl sulfate, and saponin; bile acid salts such as glycocholic acid, deoxycholic acid, and taurocholic acid; chelating agents such as EDTA and salicylic acid; fatty acids such as caproic acid, capric acid, lauric acid, oleic acid, linoleic acid, and mixed micelles; and an enamine derivative, an N-acyl collagen peptide, an N-acyl amino acid, cyclodextrins, chitosans, a nitric oxide donor, and the like.

The pill or the tablet may be coated with a sugar coating, a gastric-soluble substance, or an enteric substance.

The injection agent may contain distilled water for injection, physiological saline, propylene glycol, polyethylene glycol, a vegetable oil, alcohols, and the like. Further, a wetting agent, an emulsifying agent, a dispersing agent, a stabilizer, a solvent, a dissolution assisting agent, a preservative, and the like may be added.

The pharmaceutical composition according to the present invention may be administered in combination with other medicines or therapies that are useful for the above-described diseases.

In a case where the pharmaceutical composition according to the present invention is administered to a mammal (for example, a human, a monkey, or the like), particularly to a human, the administration dose thereof differs depending on symptoms, the age, sex, and body weight of the patient, the difference in sensitivity, the method of administration, the interval of administration, the kind of active ingredient, and the kind of formulation, and is not particularly limited. However, for example, 30 $\mu$g to 1,000 mg, 100 $\mu$g to 500 mg, or 100 $\mu$g to 100 mg can be administered once or dividedly several times. In a case of injection administration, 1 $\mu$g/kg to 3,000 $\mu$g/kg or 3 $\mu$g/kg to 1,000 $\mu$g/kg may be administered once or dividedly several times depending on the body weight of the patient.

Further, the present invention also provides:

a treatment method including administering an effective amount of the artificial protein according to the present invention to a patient having the above-described disease;
the artificial protein according to the present invention for use in the treatment or prevention of the above-described disease;
the use of the artificial protein according to the present invention for the manufacture of a pharmaceutical composition that is used in the treatment or prevention of the above-described disease; and
a therapeutic or prophylactic agent for the above-described disease, which contains the artificial protein according to the present invention.

Here, the artificial protein according to the present invention may include any one of the above-described aspects.

In the above-described aspects described in the present specification, all aspects including the aspects described in the present specification and the aspects described as the preferred aspects and the like in the present specification can be adopted as aspects in any combination thereof.

Examples

The present invention will hereinafter be described in detail by Examples and Comparative Examples. The present invention is not limited by the following examples.

[Preparation of artificial protein]

A protein having a hinge region and an Fc region of human IgG1 (SEQ ID NO: 3), amino acid sequences of which are shown in Figure 2, or a protein having an amino acid sequence set forth in SEQ ID NO: 12, in which a mutation that reduces or deletes the binding of the Fc receptor and/or the effector function of the Fc region has been introduced into the hinge region and the Fc region of the human IgG1, was used as a scaffold protein.

In addition, as the insertion sequence, the following amino acid sequences set forth in the SEQ ID NOs: 17 to 20 were used. It is noted that the SEQ ID NO: 17 corresponds to the amino acid sequence set forth in the SEQ ID NO: 1, and the SEQ ID NOs: 18 and 20 are a partial sequence of a cyclic peptide that functions as a human Met agonist, which were inserted into a protein in International Publication No. WO2019/026920, International Publication No. WO2020/027224, and International Publication No. WO2019/216436.The SEQ ID NO: 19 is an amino acid sequence in which Cys is introduced at both terminals of the amino acid sequence of SEQ ID NO: 18, and in the SEQ ID NO: 19, the Cys at both terminals are bonded to each other by a disulfide bond.

SGGYPTFYYNYRSEWVLTSGGS (SEQ ID NO: 17)
YRQFNRRTHEVWNLD (SEQ ID NO: 18)
CYRQFNRRTHEVWNLDC (SEQ ID NO: 19)
WYYAWDQTYKAFP (SEQ ID NO: 20)

DKTHTCPPCPAPE**AA**GGPSVFLFPPKPKDTLMISRTPEVTCVVV
DVSHEDPEVKFNWYVDGVEVHNAKTKPREEQYNSTYRVVSVLT
VLHQDWLNGKEYKCKVSNKAL**G**APIEKTISKAKGQPREPQVYTL
PPSRDELTKNQVSLTCLVKGFYPSDIAVEWESNGQPENNYKTTP (SEQ ID NO: 12)
PVLDSDGSFFLYSKLTVDKSRWQQGNVFSCSVMHEALHNHYTQ
KSLSLSPGK

The amino acid sequences set forth in the SEQ ID NOs: 17 to 20 were inserted into the sites corresponding to the B2 site or the B3 site of Figure 1 in the amino acid sequences set forth in the SEQ ID NOs: 3 and 12 by bonding an appropriate linker to the N-terminal and C-terminal of the amino acid sequences.

The amino acid sequences of the artificial proteins used in the present examples are shown in the table below. In the following table, "0D09", "aMD4", "aMD4ds", and "aMD5" mean that the amino acid sequences of SEQ ID NOs: 17 to 20 were inserted respectively. "B2" and "B3" indicate sites at which the amino acid sequences set forth in the SEQ ID NOs: 17 to 20 were inserted respectively. "-variant" means that a protein set forth in the SEQ ID NO: 12 was used as the scaffold protein, and for the others, the protein set forth in the SEQ ID NO: 3 was used.

[Table 1-1]

| No | amino acid sequence | SEQ ID NO |
|---|---|---|
| Fc(0D09)B2 | DKTHTCPPCPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTISK AKGQPREPQVYTLPPSRDELTKNQVSLTCLVKGFYPSDIA VEWES**SGGYPTFYYNRSEWVLTSGGS**GQPENNYKTTP PVLDSDGSFFLYSKLTVDKSRWQQGNVFSCSVMHEALH NHYTQKSLSLSPGK | 15 |
| Fc(0D09)B3 | DKTHTCPPCPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSR WQQ**SGGYPTFYYNRSEWVLTSGGS**NVFSCSVMHEAL HNHYTQKSLSLSPGK | 16 |
| Fc(0D09)B2 -variant | DKTHTCPPCPAPE**AA**GGPSVFLFPPKPKDTLMISRTPEV TCVVVDVSHEDPEVKFNWYVDGVEVHNAKTKPREEQYN STYRVVSVLTVLHQDWLNGKEYKCKVSNKAL**G**APIEKTIS KAKGQPREPQVYTLPPSRDELTKNQVSLTCLVKGFYPSDI AVEWES**SGGYPTFYYNRSEWVLTSGGS**GQPENNYKTT PPVLDSDGSFFLYSKLTVDKSRWQQGNVFSCSVMHEAL HNHYTQKSLSLSPGK | 13 |
| Fc(aMD4)B2 | DKTHTCPPCPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTISK AKGQPREPQVYTLPPSRDELTKNQVSLTCLVKGFYPSDIA VEWES**GGYRQFNRRTHEVWNLDGG**GQPENNYKTTPPV LDSDGSFFLYSKLTVDKSRWQQGNVFSCSVMHEALHNH YTQKSLSLSPGK | 21 |

[Table 1-2]

| No | amino acid sequence | SEQ ID NO |
|---|---|---|
| Fc(aMD4)B3 | DKTHTCPPCPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSR WQQ**GGYRQFNRRTHEVWNLDGG**NVFSCSVMHEALHN HYTQKSLSLSPGK | 22 |

(continued)

| No | amino acid sequence | SEQ ID NO |
|---|---|---|
| Fc(aMD4ds)B2 | DKTHTCPPCPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTISK AKGQPREPQVYTLPPSRDELTKNQVSLTCLVKGFYPSDIA VEWES**GGCYRQFNRRTHEVWNLDCGG**GQPENNYKTTP PVLDSDGSFFLYSKLTVDKSRWQQGNVFSCSVMHEALH NHYTQKSLSLSPGK | 23 |
| Fc(aMD4ds)B3 | DKTHTCPPCPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSR WQQ**GGCYRQFNRRTHEVWNLDCGG**NVFSCSVMHEAL HNHYTQKSLSLSPGK | 24 |
| Fc(aMD4ds)B3 -variant | DKTHTCPPCPAPE**AA**GGPSVFLFPPKPKDTLMISRTPEV TCVVVDVSHEDPEVKFNWYVDGVEVHNAKTKPREEQYN STYRVVSVLTVLHQDWLNGKEYKCKVSNKAL**G**APIEKTIS KAKGQPREPQVYTLPPSRDELTKNQVSLTCLVKGFYPSDI AVEWESNGQPENNYKTTPPVLDSDGSFFLYSKLTVDKSR WQQ**GGCYRQFNRRTHEVWNLDCGG**NVFSCSVMHEAL HNHYTQKSLSLSPGK | 25 |
| Fc(aMD5)B2 | DKTHTCPPCPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTISK AKGQPREPQVYTLPPSRDELTKNQVSLTCLVKGFYPSDIA VEWES**GGWYYAWDQTYKAFPGG**GQPENNYKTTPPVLD SDGSFFLYSKLTVDKSRWQQGNVFSCSVMHEALHNHYT QKSLSLSPGK | 26 |
| Fc(aMD5)B3 | DKTHTCPPCPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSR WQQ**GGWYYAWDQTYKAFPGG**NVFSCSVMHEALHNHY TQKSLSLSPGK | 27 |

Specifically, the artificial protein of Table 1 was produced as follows.

The expression of the protein was carried out using an Expi293 expression system (Thermo Fisher Scientific). The secreted protein was purified with a HiTrap Protein A HP column (Cytiva), eluted with 2 mM hydrochloric acid (pH 3.0), and neutralized with 10 x PBS. Dialysis was carried out using Slide-A-Lyzer, 2K MWCO (Thermo Fisher Scientific), and the buffer solution was substituted with PBS. As necessary, concentration was carried out using Amicon Ultra 10K (Millipore). Size exclusion chromatography was carried out using a Superdex 200 Increase 10/300 GL column (Cytiva) equilibrated with PBS, and a monodisperse fraction was purified.

[Quantification of phosphorylation level of Met in situ]

EHMES-1 human mesothelioma cells were seeded at 8,000 cells per well in a 96-well black μClear plate (Greiner

Bio-One) and cultured for 24 hours in an RPMI1640 medium to which 10% fetal bovine serum (FBS) was added. Each of the artificial proteins prepared above was added to the RPMI1640 medium to which 10% FBS was added, and the artificial protein was brought into contact with the cells for 10 minutes. Thereafter, the cells were washed with ice-cold PBS, fixed with 4% paraformaldehyde (in PBS) for 30 minutes, washed with PBS, and blocked with 5% goat serum and 0.02% Triton X-100 in PBS for 30 minutes.

The phosphorylation of Met was detected using an anti-phosphorylated Met (Tyr1234/1235) antibody (1:1,000 dilution, D26, Cell Signaling Technologies) and a horseradish peroxidase (HRP)-conjugated anti-rabbit goat antibody (1:1,000 dilution, Dako), and subsequently, the chemiluminescence by an ImmunoStar LD reagent (Wako) was measured using an ARVO MX plate reader (Perkin Elmer).

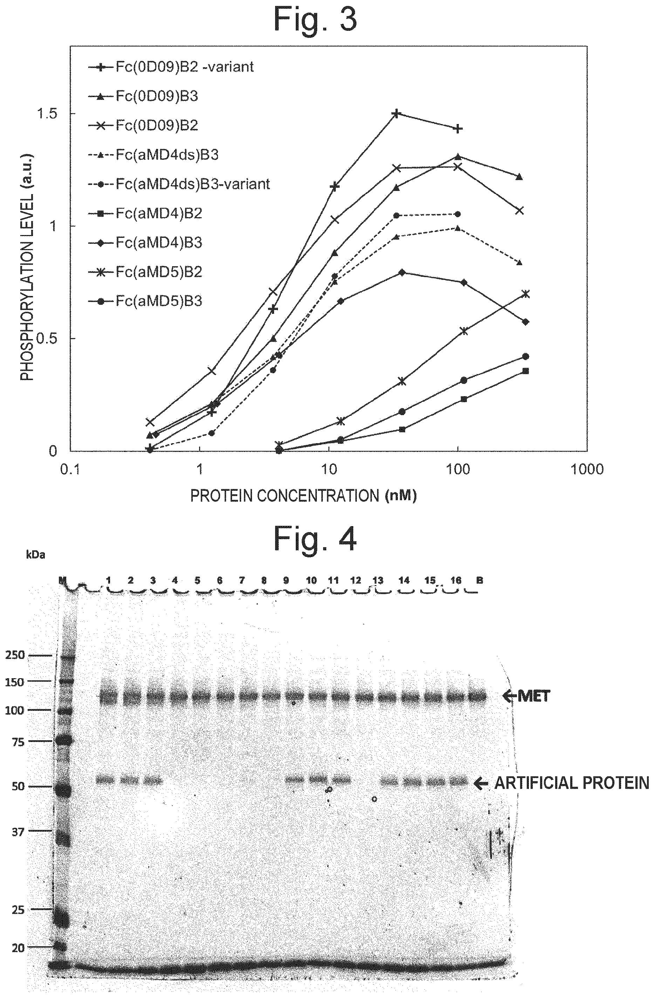

Figure 3 shows a relationship between each protein concentration and the measured phosphorylation level of Met.

Each phosphorylation level was standardized based on the value at 100 nM of Fc (aMD4ds) B3.

[Alanine scanning]

Alanine scanning was carried out for a portion of "YPTFYYNYRSEWVLTS" (SEQ ID NO: 30) in Fc (0D09) B2 having an amino acid sequence of the SEQ ID NO: 15.

First, an artificial protein in which the portion of "YPTFYYNYRSEWVLTS" (SEQ ID NO: 30) was replaced with any of the substitutions shown in the following table was produced by the same method as described above.

[Table 2]

| No. | peptide seq. | SEQ ID No. |
|---|---|---|
| 1 | YPTFYYNYRSEWVLTS | 30 |
| 2 | YATFYYNYRSEWVLTS | 31 |
| 3 | YPAFYYNYRSEWVLTS | 32 |
| 4 | YPTAYYNYRSEWVLTS | 33 |
| 5 | YPTFAYNYRSEWVLTS | 34 |
| 6 | YPTFYANYRSEWVLTS | 35 |
| 7 | YPTFYYAYRSEWVLTS | 36 |
| 8 | YPTFYYNARSEWVLTS | 37 |
| 9 | YPTFYYNYASEWVLTS | 38 |
| 10 | YPTFYYNYRAEWVLTS | 39 |
| 11 | YPTFYYNYRSAWVLTS | 40 |
| 12 | YPTFYYNYRSEAVLTS | 41 |
| 13 | YPTFYYNYRSEWALTS | 42 |
| 14 | YPTFYYNYRSEWVATS | 43 |
| 15 | YPTFYYNYRSEWVLAS | 44 |
| 16 | YPTFYYNYRSEWVLTA | 45 |

Next, a pull-down type binding assay was carried out in order to examine whether these artificial proteins have binding affinity to human Met.

A purified biotinylated extracellular region protein of human Met was used for the pull-down type binding assay. The protein containing the extracellular region of human Met was added and immobilized on Dynabeads M-280 Streptavidin (Thermo Fisher Scientific), and the beads were washed with HBS (20 mM Hepes-NaOH, 150 mM NaCl) to prepare Met-immobilized beads. After allowing a culture supernatant containing the artificial protein to react with the prepared Met-immobilized beads, the beads were washed with HBS to wash away the unbound protein, and then an SDS specimen buffer was added thereto and heating was carried out at 95°C for 5 minutes to obtain a specimen. The eluted specimen was subjected to electrophoresis under non-reducing conditions and stained with Coomassie Brilliant Blue. In this way, the artificial protein that specifically binds to the extracellular region of human Met was detected. The results are shown in Figure 4 (it is noted that the lane B shows a negative control). From Figure 4, it was found that in "YPTFYYNYRSEWVLTS" (SEQ ID NO: 30), the amino acids at positions 2 to 3, 9 to 11, and 13 to 16 are substitutable.

[Deep mutational scanning]

"YPTFYYNYRSEWVLTS" (SEQ ID NO: 30), which is an insertion sequence, was subjected to deep mutational scanning.

Specifically, the deep mutational scanning was carried out in the form of a macrocyclic peptide according to a previous report (PNAS, 2018; 115 (43): 10959-64) using the RaPID method. A DNA library, in which an appropriate codon was substituted with NNK (N's are each independently A, T, G, or C, and K is T or G) so that a peptide sequence group in which only one amino acid of SEQ ID NO: 30 was substituted with any amino acid could be synthesized, was produced. A sequence was introduced into the synthesized DNA, where the sequence was such that an HA tag for purification was added to the C-terminal.

A display library of macrocyclic peptides was produced from the mixture of the above-described DNAs by the RaPID method, and then purification by the HA tag was carried out to remove RNA to which the peptide was not bound. A part of the peptide solution recovered after the purification was aliquoted (hereinafter, referred to as "INPUT A"). The remaining was allowed to react with the above-described beads to which biotinylated human Met was bound, and then the beads were washed and subjected to a heat treatment to recover a library of peptides bound to the target (hereinafter, referred to as "INPUT B"). The libraries of INPUT A and INPUT B were amplified by PCR, and an index sequence for sequencing was further added thereto by PCR. The sequences of INPUT A and INPUT B were analyzed by a Miseq sequencer of Illumina, Inc. Next, the enrichment score was calculated as follows.

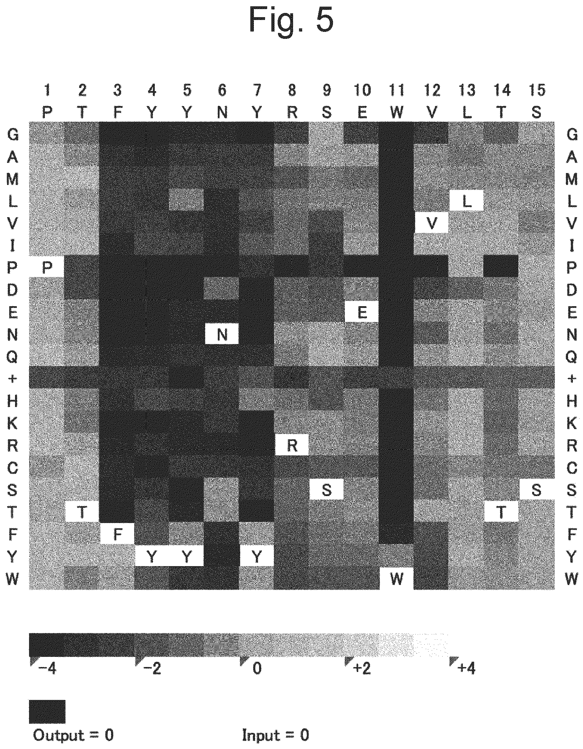

First, in order to correct the difference in the number of reads, the number of reads in INPUT A and INPUT B of each variant was divided by the total number of reads of the respective INPUTs according to the following Equation (I), whereby the ratio was calculated. Next, for each variant, the ratio before and after the reaction with respect to the target was calculated according to the following Equation (II). Then, the sequence of each variant was compared with the sequence before the mutation according to the following Equation (III), and the enrichment score was calculated by taking the logarithm with a base of 2 for the calculated ratio as shown in the following Equation (IV). A heat map was created from the enrichment score for each variant. The results are shown in Figure 5.

$$Ratio_{variant\ X,\ (INPUT\ A\ or\ INPUT\ B)} = \frac{Number\ of\ reads\ of\ variant\ X\ in\ INPUTs\ A\ and\ B}{Total\ number\ of\ reads\ in\ INPUTs\ A\ and\ B} \qquad (\mathrm{I})$$

$$\gamma_{variant\ X} = \frac{Ratio_{variant\ X,\ INPUT\ B}}{Ratio_{variant\ X,\ INPUT\ A}} \qquad (\mathrm{II})$$

$$\delta_{variant\ X} = \frac{\gamma_{variant\ X}}{\gamma_{WT}} \qquad (\mathrm{III})$$

$$Enrichment\ score = log_2 \delta_{variant\ X} \qquad (\mathrm{IV})$$

[Change of scaffold protein]

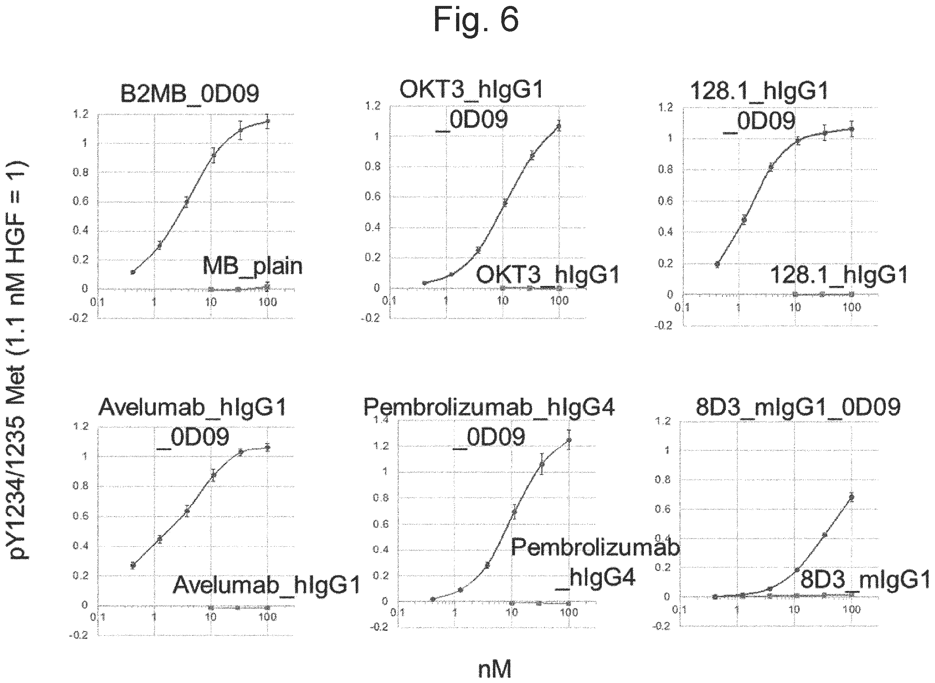

Artificial antibodies were produced by changing the scaffold protein from the hinge region and the Fc region of human IgG1 to the following antibodies and inserting "SGGYPTFYYNYRSEWVLTSGGS" (SEQ ID NO: 17) into the Fc region of the antibody according to the above-described production method of the artificial protein. The Met phosphorylation ability of each of the produced artificial antibodies was evaluated according to the same method as described above. The Met phosphorylation ability was also measured in the same manner for an antibody in which "SGGYPTFYYNYRSEWVLTSGGS" (SEQ ID NO: 17) was not inserted. The results are shown in Figure 6. It is noted that in Table 3, "B2MB_0D09" is a positive control and is an artificial protein having the same amino acid sequence as Fc (0D09) B2 in Table 1 above. In addition, in the following table, the "insertion position" indicates a position where an insertion sequence is inserted into each artificial antibody, and it indicates the corresponding portion in the Fc region derived from human IgG1 in a case where each antibody before the insertion of the insertion sequence is aligned with the Fc region derived from human IgG1 which is set forth in the SEQ ID NO: 4. That is, in each artificial antibody, the insertion sequence was inserted at a position corresponding to the B2 (SNG: positions 383 to 385) site of the Fc region derived from human IgG1 in a case where each antibody before the insertion of the insertion sequence is aligned with the Fc region derived from human IgG1 which is set forth in the SEQ ID NO: 4.

[Table 3]

| No. | Sample name | Antibody or antibody fragment | Insertion position |
|---|---|---|---|
| 1 | B2MB_0D09 | Hinge region and Fc region of human IgG | B2 |

(continued)

| No. | Sample name | Antibody or antibody fragment | Insertion position |
|---|---|---|---|
| 2 | OKT3_hIgG1_0D09 | Anti-CD3 antibody | B2 |
| 3 | 128.1_hIgG1_0D09 | Anti-human TfR antibody | B2 |
| 4 | Avelumab_hIgG1_0D09 | Avelumab (anti-human PD-L1 antibody) | B2 |
| 5 | Pembrolizumab_hIgG4_0D09 | Pembrolizumab (anti-human PD-1 antibody) | B2 |
| 6 | 8D3_mIgG1_0D09 | Anti-mouse TfR antibody | B2 |

## Claims

1. An artificial protein that comprises an Fc region of an antibody, where an amino acid sequence of SEQ ID NO: 1 is inserted into the Fc region,
$(X^1)_{n1}YX^3X^4FYYNYX^5X^6X^7WX^8X^9X^{10}X^{11}(X^2)_{n2}$ (SEQ ID NO: 1) (in the SEQ ID NO: 1, $X^1$, $X^2$, $X^3$, $X^4$, $X^5$, $X^6$, $X^7$, $X^8$, $X^9$, $X^{10}$, and $X^{11}$ each independently represent an amino acid, and n1 and n2 are each independently an integer of 0 or more and 6 or less).

2. The artificial protein according to Claim 1, wherein the amino acid sequence of the SEQ ID NO: 1 is inserted into a loop portion, wherein the loop portion is exposed on a molecular surface of the Fc region of the antibody.

3. The artificial protein according to Claim 1 or 2, wherein the amino acid sequence inserted into the Fc region is set forth in SEQ ID NO: 28,
$(X^1)_{n1}YPTFYYNYRSEWVLTS(X^2)_{n2}$ (SEQ ID NO: 28) (in the SEQ ID NO: 28, $X^1$, $X^2$, n1, and n2 are each defined in the same manner as in the SEQ ID NO: 1).

4. The artificial protein according to any one of Claims 1 to 3, wherein the Fc region of the antibody is derived from an Fc region of IgG.

5. The artificial protein according to any one of Claims 1 to 3, wherein the Fc region of the antibody is derived from an Fc region of IgG1.

6. The artificial protein according to any one of Claims 1 to 5, wherein the Fc region of the antibody is an Fc region of an antibody in which one or more amino acids are deleted, added, and/or substituted compared to an Fc region of a wild-type antibody.

7. A Met protein agonist comprising:
the artificial protein according to any one of Claims 1 to 6.

8. A pharmaceutical composition comprising:
the artificial protein according to any one of Claims 1 to 6.

9. The pharmaceutical composition according to Claim 8, wherein the pharmaceutical composition is used for treatment or prevention of a disease selected from the group consisting of acute hepatitis, fulminant hepatitis, hepatic cirrhosis, hepatic fibrosis, biliary atresia, fatty liver, non-alcoholic steatohepatitis, alcoholic steatohepatitis, acute renal failure, chronic kidney disease, diabetic nephropathy, acute pneumonia, pulmonary fibrosis, angiopathy, myocardial infarction, dilated cardiomyopathy, skin ulcer, cerebral infarction, amyotrophic lateral sclerosis, glomerular nephritis, chronic allograft nephropathy, obstructive arteriosclerosis, critical limb ischemia, pulmonary emphysema, asthma, and vocal cord scarring.

# Fig. 1

# Fig. 2

```
221 D K T H T C P P C P A P E L L G G P S V 240
241 F L F P P K P K D T L M I S R T P E V T 260
261 C V V V D V S H E D P E V K F N W Y V D 280
281 G V E V H N A K T K P R E E Q Y N S T Y 300
301 R V V S V L T V L H Q D W L N G K E Y K 320
321 C K V S N K A L P A P I E K T I S K A K 340
341 G Q P R E P Q V Y T L P P S R D E L T K 360
361 N Q V S L T C L V K G F Y P S D I A V E 380
381 W E S N G Q P E N N Y K T T P P V L D S 400
401 D G S F F L Y S K L T V D K S R W Q Q G 420
421 N V F S C S V M H E A L H N H Y T Q K S 440
441 L S L S P G K
```

# Fig. 3

# Fig. 4

# Fig. 5

## Fig. 6

EP 4 582 457 A1

# INTERNATIONAL SEARCH REPORT

| International application No. |
|---|
| **PCT/JP2023/031626** |

| A. | CLASSIFICATION OF SUBJECT MATTER |
|---|---|

*C07K 19/00*(2006.01)i; *A61K 38/00*(2006.01)i; *A61K 47/68*(2017.01)i; *A61P 1/16*(2006.01)i; *A61P 3/06*(2006.01)i; *A61P 9/00*(2006.01)i; *A61P 9/10*(2006.01)i; *A61P 9/14*(2006.01)i; *A61P 11/00*(2006.01)i; *A61P 11/06*(2006.01)i; *A61P 13/12*(2006.01)i; *A61P 17/00*(2006.01)i; *A61P 21/00*(2006.01)i; *A61P 37/06*(2006.01)i; *C07K 7/08*(2006.01)i; *C07K 16/00*(2006.01)i
FI: C07K19/00 ZNA; C07K16/00; C07K7/08; A61K38/00; A61P1/16; A61P3/06; A61P9/00; A61P9/10; A61P9/10 101; A61P9/14; A61P11/00; A61P11/06; A61P13/12; A61P17/00; A61P21/00; A61P37/06; A61K47/68

According to International Patent Classification (IPC) or to both national classification and IPC

| B. | FIELDS SEARCHED |
|---|---|

Minimum documentation searched (classification system followed by classification symbols)

C07K19/00; A61K38/00; A61K47/68; A61P1/16; A61P3/06; A61P9/00; A61P9/10; A61P9/14; A61P11/00; A61P11/06; A61P13/12; A61P17/00; A61P21/00; A61P37/06; C07K7/08; C07K16/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | WO 2019/026920 A1 (THE UNIVERSITY OF TOKYO, OSAKA UNIVERSITY) 07 February 2019 (2019-02-07) abstract, claims, examples, fig. 1, 8, paragraph [0063] | 1-9 |
| Y | WO 2020/027224 A1 (THE UNIVERSITY OF TOKYO) 06 February 2020 (2020-02-06) abstract, claims, examples, paragraphs [0051], [0054], fig. 1-3 | 1-9 |
| Y | ITO, K. et al. Artificial human Met agonists based on macrocycle scaffolds. Nat. Commun. 11 March 2015, vol. 6, no. 6373, pp. 1-12, pp. 2-26 of Supply Info. abstract, supply table 1 | 1-9 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 November 2023** | **14 November 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

International application No.

**PCT/JP2023/031626**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed:

       ☑ in the form of an Annex C/ST.25 text file.

       ☐ on paper or in the form of an image file.

   b. ☐ furnished together with the international application under PCT Rule 13ter.1(a) for the purposes of international search only in the form of an Annex C/ST.25 text file.

   c. ☐ furnished subsequent to the international filing date for the purposes of international search only:

       ☐ in the form of an Annex C/ST.25 text file (Rule 13ter.1(a)).

       ☐ on paper or in the form of an image file (Rule 13ter.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "In the form of an Annex C/ST.25 text file" above should be understood as "in ST. 26 format".

Form PCT/ISA/210 (continuation of first sheet) (January 2015)

## INTERNATIONAL SEARCH REPORT
### Information on patent family members

International application No.

**PCT/JP2023/031626**

| Patent document<br>cited in search report | Publication date<br>(day/month/year) | Patent family member(s) | Publication date<br>(day/month/year) |
|---|---|---|---|
| WO 2019/026920 A1 | 07 February 2019 | US 2020/0377588 A1<br>abstract, claims, examples, fig.<br>1, 8<br>EP 3666897 A1<br>KR 10-2020-0032184 A<br>CN 110997921 A | |
| WO 2020/027224 A1 | 06 February 2020 | US 2021/0317233 A1<br>abstract, claims, paragraphs<br>[0166], [0167], fig. 1-3<br>EP 3831846 A1<br>CN 112566928 A<br>KR 10-2021-0038580 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20120251531 A
- US 9505826 B
- US 20140056879 A
- US 20120107871 A
- US 20190300621 A
- WO 2019026920 A
- WO 2020027224 A
- WO 2019216436 A

### Non-patent literature cited in the description

- **TRUSOLINO, L.** ; **BERTOTTI, A** ; **COMOGLIO, P.M.** MET signalling: principles and functions in development, organ regeneration and cancer. *Nat. Rev. Mol. Cell. Biol.*, 2010, vol. 11, 834-848
- **SAKAI, K.** ; **AOKI, S** ; **MATSUMOTO, K.** Hepatocyte growth factor and Met in drug discovery. *J. Biochem.*, 2015, vol. 157, 271-284
- **DESOLE, C. et al.** HGF and MET: From brain development to neurological disorders.. *Front Cell Dev Biol.*, 2021, vol. 9, 683609
- **HIRANO, S. et al.** A phase I/II exploratory clinical trial for intracordal injection of recombinant hepatocyte growth factor for vocal fold scar and sulcus. *J Tissue Eng Regen Med.*, 2018, vol. 12, 1031-1038
- **NAGOSHI, N. et al.** Phase I/II Study of intrathecal administration of recombinant human hepatocyte growth factor in patients with acute spinal cord injury: A double-blind, randomized clinical trial of safety and efficacy. *J Neurotrauma.*, 2020, vol. 37, 1752-1758
- **IDO, A. et al.** Safety and pharmacokinetics of recombinant human hepatocyte growth factor (rh-HGF) in patients with fulminant hepatitis: a phase I/II clinical trial, following preclinical studies to ensure safety. *J Transl Med.*, 2011, vol. 9 (55)
- **LI, N. et al.** Therapeutic effect of HGF on NASH mice through HGF/c-Met and JAK2-STAT3 signalling pathway.. *Ann Hepatol.*, 2018, vol. 17, 501-510
- **YANG, Y.M. et al.** Interventional potential of recombinant feline hepatocyte growth factor in a mouse model of non-alcoholic steatohepatitis.. *Front Endocrinol.*, 2018, vol. 9, 378
- **MATSUDA, Y. et al.** Hepatocyte growth factor prevents liver cirrhosis caused by dimethylnitrosamine in rats. *J Biochem.*, 1995, vol. 118, 643-649
- **MIZUNO, S. et al.** Hepatocyte growth factor prevents renal fibrosis and dysfunction in a mouse model of chronic renal disease. *J Clin Invest.*, 1998, vol. 101, 1827-1834
- **KAWAIDA, K. et al.** Hepatocyte growth factor prevents acute renal failure and accelerate renal regeneration in mice. *Proc Natl Acad Sci USA.*, 1994, vol. 91, 4357-4361
- **KOSAI, K.** Hepatocyte growth factor abrogates Fas-induced lethal liver apoptosis in mice. *Biochem Biophys Res Commun.*, 1998, vol. 244, 683-690
- **TAHARA, M. et al.** Hepatocyte growth factor leads to recovery from alcohol-induced fatty liver in rats. *J Clin Invest.*, 1999, vol. 103, 313-320
- **YAEKASHIWA, M. et al.** Simultaneous or delayed administration of hepatocyte growth factor (HGF) equally repress the fibrotic change in murine lung injury induced by bleomycin: A morphologic study.. *Am J Resp & Crit Care Med.*, 1997, vol. 156, 1937-1944
- **NAKAMURA, T. et al.** Hepatocyte growth factor prevents tissue fibrosis, remodeling, and dysfunction in cardiomyopathic hamster hearts.. *Am J Physiol.*, 2005, vol. 288, H2131-H2139
- **AZUMA, H. et al.** Hepatocyte growth factor prevents development of chronic allograft nephropathy in an experimental rat transplant model. *J Am Soc Nephrol.*, 2001, vol. 12, 1280-1292
- **ITO, W. et al.** Growth factors temporally associate with airway responsiveness and inflammation in allergen-exposed mice.. *Int Arch Allergy Immunol.*, 2008, vol. 145, 324-339
- **OHMICHI, H.** ; **MATSUMOTO, K.** ; **NAKAMURA, T.** In vivo mitogenic action of hepatocyte growth factor on lung epithelial cells: pulmotrophic role in lung regeneration. *Am J Physiol.*, 1996, vol. 270, L1031-L1039
- **DATE, I. et al.** Hepatocyte growth factor attenuates cerebral ischemia-induced learning dysfunction. *Biochem Biophys Res Commun.*, 2004, vol. 319, 1152-1158
- **INAMPUDI, C. et al.** Angiogenesis in peripheral arterial disease.. *Curr Opin Pharmacol.*, 2018, vol. 39, 60-67

- *PNAS*, 2018, vol. 115 (43), 10959-64